# EUROPEAN PATENT SPECIFICATION

(11) **EP 3 427 655 B1**
(45) Date of publication and mention of the grant of the patent: **10.03.2021**
(21) Application number: 17782509.8
(22) Date of filing: 14.04.2017
(51) Int. Cl.: A61B 5/021, A61B 5/022, A61B 5/08, A61B 5/145, A61B 5/00, A61B 5/11

(54) **BIOLOGICAL INFORMATION ANALYZING DEVICE, SYSTEM, AND PROGRAM**
VORRICHTUNG, SYSTEM UND PROGRAMM ZUR ANALYSE BIOLOGISCHER INFORMATIONEN
DISPOSITIF, SYSTÈME ET PROGRAMME D'ANALYSE D'INFORMATIONS BIOLOGIQUES

(30) Priority: 15.04.2016 JP 2016082463
(43) Date of publication of application: 16.01.2019
(73) Proprietor: Omron Corporation, Kyoto-shi, Kyoto 600-8530 (JP); Omron Healthcare Co., Ltd., Muko-shi, Kyoto 617-0002 (JP)
(72) Inventor: NAKAJIMA, Hiroshi, Kyoto-shi Kyoto 600-8530 (JP); WADA, Hirotaka, Kyoto-shi Kyoto 600-8530 (JP); TSUCHIYA, Naoki, Kyoto-shi Kyoto 600-8530 (JP); KASAI, Masaaki, Kyoto-shi Kyoto 600-8530 (JP); KAN, Eriko, Kyoto-shi Kyoto 600-8530 (JP); UENOYAMA, Toru, Tokyo 108-0075 (JP); OBAYASHI, Keiichi, Kyoto-shi Kyoto 600-8530 (JP); KOKUBO, Ayako, Kyoto-shi Kyoto 600-8530 (JP); OTA, Yuya, Kyoto-shi Kyoto 600-8530 (JP); SHIGA, Toshikazu, Muko-shi Kyoto 617-0002 (JP); KUWABARA, Mitsuo, Muko-shi Kyoto 617-0002 (JP); SATO, Hironori, Muko-shi Kyoto 617-0002 (JP); MIYAGAWA, Ken, Muko-shi Kyoto 617-0002 (JP); TSUTSUMI, Masakazu, Muko-shi Kyoto 617-0002 (JP)
(74) Representative: Isarpatent
(86) International application number: PCT/JP2017/015277
(87) International publication number: WO 2017/179696

(56) References cited:
- EP-A1- 0 872 255
- WO-A1-2014/171465
- JP-A- H09 220 207
- JP-A- 2002 536 104
- JP-A- 2005 532 111
- JP-A- 2006 212 218
- JP-A- 2008 086 568
- JP-A- 2008 536 545
- JP-A- 2011 189 080
- JP-A- 2014 000 105
- US-A1- 2011 190 643
- US-A1- 2013 053 664

## Description

### TECHNICAL FIELD

The present invention relates to a technique for obtaining useful information from a measured blood pressure waveform.

### RELATED ART

Techniques are known in which changes in the internal pressure of the radial artery are measured and the shape of pressure pulses (a blood pressure waveform) is recorded. Patent Document 1 (JP 2008-61824A) discloses measuring a blood pressure waveform through tonometry and obtaining information such as AI (Augmentation Index) value, pulse wave period, baseline fluctuation rate, sharpness, ET (Ejection Time), and the like from the blood pressure waveform. Patent Document 2 (JP 2005-532111A), meanwhile, discloses measuring a blood pressure waveform using a wristwatch-type blood pressure monitor, calculating a mean arterial pressure, a mean systolic pressure, a mean diastolic pressure, a mean systolic pressure index, and a mean diastolic pressure index from the blood pressure waveform, and then outputting an alert when those values deviate from reference values.

### RELATED ART DOCUMENTS

### PATENT DOCUMENTS

Patent Document 1: JP 2008-61824A
Patent Document 2: JP 2005-532111A
   EP 0 872 255 A1 describes a relax guiding device and a biofeedback guiding device.
   US 2011/0190643 A1 describes a system for cardiac status determination. The system improves detection and diagnosis of blood pressure based cardiac function.

### SUMMARY OF THE INVENTION

### PROBLEM TO BE SOLVED BY THE INVENTION

The inventors of the present invention are undertaking diligent research toward putting into practical use blood pressure measurement devices capable of accurately measuring a blood pressure waveform for each heartbeat during free movement. Through experiments using test subjects throughout the course of this development, the inventors of the present invention discovered that many types of useful information can be extracted from blood pressure waveform data measured continuously during free movement.

Blood pressure fluctuations include a variety of types of fluctuations, including long-term fluctuations arising with age, fluctuations depending on the day of the week, fluctuations over the course of a day, fluctuations caused by environment changes (e.g., temperature changes), and fluctuations caused by changes in a user's state (e.g., physiological changes such as the occurrence of sleep apnea, physical changes such as exercise and standing, and so on). The inventors of the present invention discovered that of these fluctuations, fluctuations caused by environment changes and changes in the user's state manifest as blood pressure fluctuations in different ways depending on that person's condition, risks, attributes (age, sex, and so on), and the like. Conventional blood pressure monitors can only measure blood pressure during rest or measure mean values such as mean systolic pressure, and it has thus been difficult to accurately detect instantaneous fluctuations in blood pressure caused by environment changes, changes in a user's state, and so on.

Accordingly, an object of the present invention is to provide a novel technique for evaluating the way in which blood pressure fluctuations appear from user to user. A further object of the present invention is to provide a novel technique for estimating a user's risk on the basis of the way in which the blood pressure fluctuates.

### MEANS FOR SOLVING THE PROBLEMS

To achieve the above-described object, the present invention employs the following configurations.

A biological information analyzing device according to the present invention is a biological information analyzing device including: an indicator extraction unit configured to obtain data of a blood pressure fluctuation section, which is a section in which a blood pressure fluctuation has occurred, from time series data of a blood pressure waveform measured continuously by a sensor, which is configured to be worn on a user's body and can non-invasively measure a blood pressure waveform for each of a plurality of heartbeats, and extract an indicator expressing a blood pressure fluctuation type of the user from the data of the blood pressure fluctuation section; and a processing unit configured to output the indicator expressing the blood pressure fluctuation type extracted by the indicator extraction unit. According to this configuration, the user's blood pressure fluctuation type is determined on the basis of measurement data in a section where the user's blood pressure has fluctuated, and that information is then output. Accordingly, the user can quickly take measures appropriate to his/her own characteristics. If the device is used by a doctor, a nurse, or the like, that person can refer to the user's (patient's) blood pressure fluctuation type, and then carry out treatment, guidance, and so on appropriate for the user's characteristics.

According to the invention, the indicator extraction unit is configured to calculate time series data of a systolic blood pressure for each heartbeat from the time series data of the blood pressure waveform, and extract, as the data of the blood pressure fluctuation section, data of a period between two minimum points on either side of a maximum point in the time series data of the systolic blood pressure. Using this extraction method makes it possible to detect the blood pressure fluctuation section simply and automatically.

Preferably, the indicator extraction unit is configured to extract a blood pressure fluctuation feature amount from the data of the blood pressure fluctuation section, and take a trend in the extracted blood pressure fluctuation feature amount as one indicator expressing the blood pressure fluctuation type. This is because the way in which a blood pressure fluctuation appears is thought to be related to the user's characteristics, risk, and so on. Additionally, it is preferable that the indicator extraction unit estimate a cause of the blood pressure fluctuation in the blood pressure fluctuation section, and take the estimated cause as one indicator expressing the blood pressure fluctuation type. This is because the cause of a blood pressure fluctuation is also thought to be related to the user's characteristics, risk, and so on. Preferably, the indicator extraction unit is configured to comprehensively evaluate trends in the blood pressure fluctuation feature amounts and the cause of the blood pressure fluctuation. This can be expected to make it possible to more accurately estimate the user's characteristics, risk, and so on.

For example, preferably, the indicator extraction unit is configured to classify the user's blood pressure fluctuation feature amount by referring to a class database in which trends of the blood pressure fluctuation feature amount are registered as a plurality of different groups and determine whether the blood pressure fluctuation feature amount extracted from the data of the blood pressure fluctuation section conforms to the trend of the blood pressure fluctuation feature amount in any of the plurality of groups.

For example, preferably, the indicator extraction unit is configured to estimate the cause of the blood pressure fluctuation in the blood pressure fluctuation section on the basis of information pertaining to a state of the user detected at a time corresponding to the blood pressure fluctuation section by a second sensor configured to detect the state of the user.

Preferably, the indicator extraction unit is configured to obtain risk information corresponding to the user's blood pressure fluctuation type from a risk database in which the blood pressure fluctuation type is associated with the risk information, and the processing unit is configured to output the risk information obtained by the indicator extraction unit. According to this configuration, the user's blood pressure fluctuation type is determined on the basis of measurement data in a section where the user's blood pressure has fluctuated, and risk information corresponding to the blood pressure fluctuation type is then output. Accordingly, the user can quickly take measures appropriate to his/her own risk. If the device is used by a doctor, a nurse, or the like, that person can refer to the risk corresponding to the user's (patient's) blood pressure fluctuation type, and then carry out treatment, guidance, and so on appropriate for the user's risk.

A biological information analyzing system according to the present invention is a biological information analyzing system including: a sensor, which is configured to be worn on a user's body and can non-invasively measure a blood pressure waveform for each of heartbeats; and a biological information analyzing device as described above configured to analyze biological information using data of the blood pressure waveform measured continuously by the sensor.

A program according to the present invention is a program causing a processor to function as the indicator extraction unit and the processing unit of the biological information analyzing device described above.

The present invention can also be realized as a biological information analyzing method as defined in claim 11.

### EFFECTS OF THE INVENTION

According to the present invention, a novel technique for evaluating the way in which blood pressure fluctuations appear from user to user can be provided. Additionally, a novel technique for estimating a user's risk on the basis of the way in which the blood pressure fluctuates can be provided.

### BRIEF DESCRIPTION OF THE DRAWINGS

Fig. 1 is a diagram illustrating the external appearance of the overall configuration of a biological information analyzing system 10.
Fig. 2 is a block diagram illustrating the hardware configuration of the biological information analyzing system 10.
Fig. 3 is a cross-sectional view schematically illustrating the structure of a blood pressure measurement unit 20 and a measurement state.
Fig. 4 is a diagram illustrating a blood pressure waveform measured by the blood pressure measurement unit 20.
Fig. 5 is a block diagram illustrating processing performed by a biological information analyzing device 1.
Fig. 6 is a diagram illustrating the waveform of a pressure pulse (a blood pressure waveform) in the radial artery, for a single heartbeat.
Fig. 7 is a flowchart illustrating an event occurrence risk estimation process according to Example 1.
Fig. 8 is a diagram illustrating an example of a blood pressure fluctuation section extraction method according to Example 1.
Figs. 9A to 9D are diagrams illustrating an example of waveform feature amounts according to Example 1.
Fig. 10 is a diagram illustrating an example of information pertaining to a user's blood pressure fluctuation section according to Example 1.
Fig. 11 is a diagram conceptually illustrating a process for classifying blood pressure fluctuation features according to Example 1.
Fig. 12 is a diagram illustrating an example of a risk database according to Example 1.
Fig. 13 is an example of an information display screen according to Example 1.

### EMBODIMENTS OF THE INVENTION

A preferred embodiment of the present invention will be described below with reference to the drawings. Note, however, that the descriptions of configurations given hereinafter should be changed as appropriate depending on the configuration of the device to which the invention is applied, various types of conditions, and so on, and the scope of the invention is not intended to be limited by the following descriptions.

### Biological Information Analyzing System

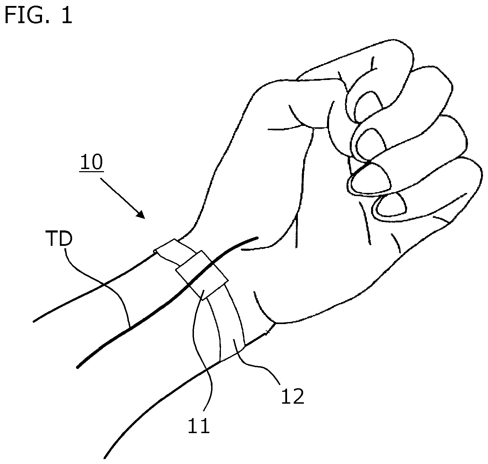

Fig. 1 is a diagram illustrating the external appearance of the overall configuration of a biological information analyzing system 10 according to an embodiment of the present invention. Fig. 1 illustrates a state in which the biological information analyzing system 10 is worn on the left wrist. The biological information analyzing system 10 includes a main unit 11 and a belt 12 fixed to the main unit 11. The biological information analyzing system 10 is what is known as a wearable device, and is worn so that the main unit 11 is in contact with the skin on the inner side of the wrist and so that the main unit 11 is arranged above a radial artery TD located beneath the skin. Although the present embodiment describes a configuration in which the device is worn above the radial artery TD, the configuration may be such that the device is worn above another superficial artery.

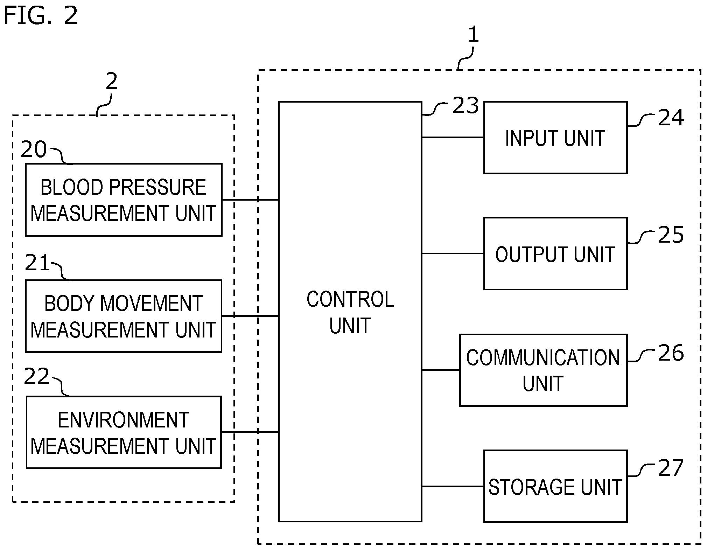

Fig. 2 is a block diagram illustrating the hardware configuration of the biological information analyzing system 10. Broadly speaking, the biological information analyzing system 10 includes a measurement unit 2 and a biological information analyzing device 1. The measurement unit 2 is a device that obtains information used to analyze biological information through measurement, and includes a blood pressure measurement unit 20, a body movement measurement unit 21, and an environment measurement unit 22. However, the configuration of the measurement unit 2 is not limited to that illustrated in Fig. 2. For example, units that measure biological information aside from blood pressure and body movement (body temperature, blood sugar, brain waves, and so on) may be added. Alternatively, units not used in the Example described later are not required configurations and may therefore be omitted from the biological information analyzing system 10. The biological information analyzing device 1 is a device that analyzes biological information on the basis of information obtained from the measurement unit 2, and includes a control unit 23, an input unit 24, an output unit 25, a communication unit 26, and a storage unit 27. The units 20 to 27 are connected to each other by a local bus and other signal lines so as to be capable of exchanging signals. The biological information analyzing system 10 also includes a power source (a battery), which is not illustrated.

The blood pressure measurement unit 20 is a unit that measures pressure pulses in the radial artery TD through tonometry. Tonometry is a non-invasive method of measuring pressure pulses using a pressure sensor, in which an artery is compressed from above the skin at an appropriate pressure to form a flat part in an artery TD, and the internal pressure and external pressure of the artery are balanced.

The body movement measurement unit 21 is a unit, including a three-axis accelerometer, that measures movement of a user's body (body movement) using the accelerometer. The body movement measurement unit 21 may include a circuit that converts the output of the three-axis accelerometer into a format that can be read by the control unit 23.

The environment measurement unit 22 is a unit that measures environment information that can affect the user's physical/mental state (and blood pressure in particular). The environment measurement unit 22 can include a temperature sensor, a humidity sensor, an illuminance sensor, an altitude sensor, a location sensor, and the like, for example. The environment measurement unit 22 may include a circuit that converts the output of these sensors into a format that can be read by the control unit 23.

The control unit 23 is a unit that handles a variety of processes, such as controlling the various parts of the biological information analyzing system 10, acquiring data from the measurement unit 2, storing the acquired data in the storage unit 27, processing/analyzing the data, inputting/outputting the data, and so on. The control unit 23 includes a hardware processor (called a CPU hereinafter), ROM (Read-Only Memory), RAM (Random Access Memory), and the like. The processing carried out by the control unit 23, which will be described later, is realized by the CPU reading programs stored in the ROM or the storage unit 27 and executing those programs. The RAM functions as a work memory when the control unit 23 carries out various types of processes. Although the present embodiment describes a configuration in which the control unit 23 acquires the data from the measurement unit 2 and stores the data in the storage unit 27, the configuration may be such that the data is stored (written) directly from the measurement unit 2 into the storage unit 27.

The constituent elements of the embodiment, e.g., the measurement units, an indicator extraction unit, a processing unit, a determination unit, a risk database, the input unit, the output unit, a case database, and the like may be provided as hardware in the biological information analyzing system 10. The indicator extraction unit, the processing unit, and the determination unit may receive executable programs stored in the storage unit 27 and execute those programs. The indicator extraction unit, the processing unit, and the determination unit may receive data from the blood pressure measurement unit 20, the body movement measurement unit 21, the environment measurement unit 22, the input unit 24, the output unit 25, the communication unit 26, the storage unit 27, and so on as necessary. Databases such as the risk database and the case database may be provided in the storage unit 27 or the like, and may store information arranged so that the data can be searched and accumulated with ease. The structure, operations, and the like of the biological information analyzing system 10 are disclosed in JP 2016-082069, for example. The structure, operations, and the like of the blood pressure measurement unit are disclosed in JP 2016-087003A.

The input unit 24 is a unit that provides an operation interface to the user. For example, operation buttons, switches, a touch panel, or the like can be used.

The output unit 25 is a unit that provides, to the user, an interface that outputs information. For example, a display device that outputs information as images (a liquid crystal display or the like), an audio output device or a buzzer that outputs information as audio, an LED that outputs information by emitting or extinguishing light, a vibration device that outputs information as vibrations, or the like can be used.

The communication unit 26 is a unit that carries out data communication with other devices. Any system, including wireless LAN, Bluetooth (registered trademark), or the like, may be used as the data communication system.

The storage unit 27 is a storage medium in which data can be stored and from which data can be read out, and stores programs executed by the control unit 23, measurement data obtained from the measurement units, various types of data obtained by processing the measurement data, and so on. The storage unit 27 is a medium that electrically, magnetically, optically, mechanically, or chemically stores the information to be stored. Flash memory can be used, for example. The storage unit 27 may be a portable type such as a memory card, or may be built into the biological information analyzing system 10.

Some or all of the body movement measurement unit 21, the environment measurement unit 22, the control unit 23, the input unit 24, the output unit 25, and the storage unit 27 may be configured as devices separate from the main unit 11. In other words, as long as the main unit 11 including the blood pressure measurement unit 20 and a circuit that controls the blood pressure measurement unit 20 can be worn on a wrist, the structures of other units can be designed as desired. In this case, the main unit 11 is linked to the other units via the communication unit 26. A variety of configurations are conceivable, such as implementing the functions of the control unit 23, the input unit 24, the output unit 25, and so on as a smartphone app, or obtaining necessary data from an activity meter having the functions of the body movement measurement unit 21, the environment measurement unit 22, and so on. Sensors that measure biological information aside from blood pressure may be provided as well. For example, a sleep sensor, a pulse oximeter (SpO2 sensor), a breathing sensor (flow sensor), a blood sugar level sensor, and so on may be combined as well.

Although the present embodiment describes providing a sensor that measures blood pressure (the blood pressure measurement unit 20) and a configuration that carries out analysis processes on blood pressure waveform data (the control unit 23 and the like) in a single device, these elements may be configured as separate entities. In the present embodiment, a configuration that carries out analysis processes on biological information (the control unit 23 and the like) is called a "biological information analyzing device", and a device configured by combining a measurement unit and the biological information analyzing device is called a "biological information analyzing system". However, these names are used for the sake of simplicity, and the measurement unit and the configuration that carries out analysis processes on biological information may as a whole be called a "biological information analyzing device", or other names may be used instead.

### Blood Pressure Waveform Measurement

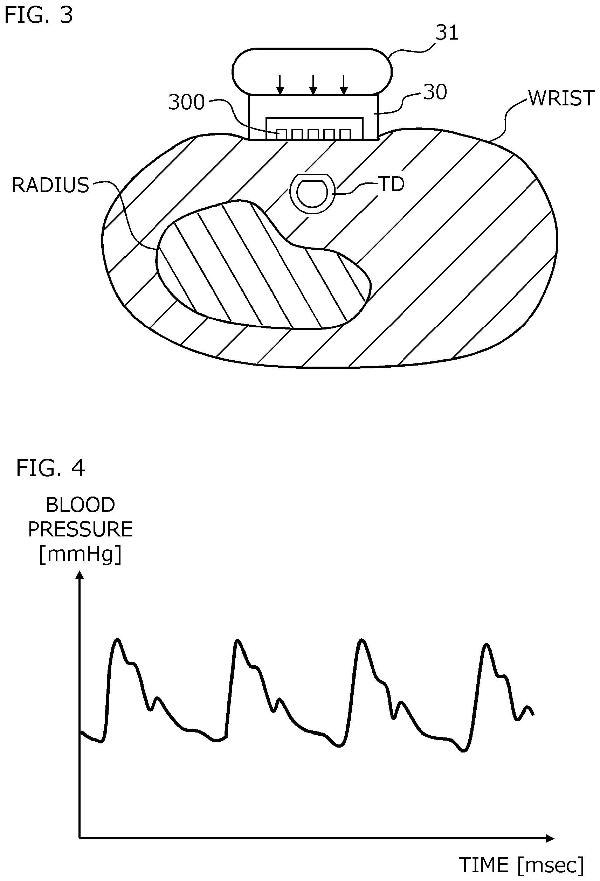

Fig. 3 is a cross-sectional view schematically illustrating the structure of the blood pressure measurement unit 20 and a measurement state. The blood pressure measurement unit 20 includes a pressure sensor 30, and a compression mechanism 31 for pressing the pressure sensor 30 against the wrist. The pressure sensor 30 includes a plurality of pressure detection elements 300. The pressure detection elements 300 are elements that detect a pressure and convert the pressure into an electrical signal, and elements that use a piezoresistance effect, for example, can be favorably used. The compression mechanism 31 is constituted by, for example, an air bladder and a pump that adjusts the internal pressure of the air bladder. When the control unit 23 controls the pump to increase the internal pressure of the air bladder, the air bladder expands and presses the pressure sensor 30 against the surface of the skin. Note that the compression mechanism 31 may be any mechanism capable of adjusting a compressive force of the pressure sensor 30 against the surface of the skin, and is not limited to a mechanism employing an air bladder.

When the biological information analyzing system 10 is secured to the wrist and started, the control unit 23 controls the compression mechanism 31 of the blood pressure measurement unit 20 to keep the compressive force of the pressure sensor 30 in an appropriate state (a tonometry state). Pressure signals detected by the pressure sensor 30 are then acquired sequentially by the control unit 23. The pressure signals obtained by the pressure sensor 30 are generated by taking analog physical amounts (e.g., voltage values) outputted by the pressure detection elements 300 and digitizing those physical amounts through an A/D conversion circuit or the like that employs a known technique. Suitable analog values such as current values, resistance values, or the like may be employed as the analog physical amounts, depending on the types of the pressure detection elements 300. The signal processing such as A/D conversion may be carried out by providing a predetermined circuit in the blood pressure measurement unit 20, or may be carried out by another unit (not shown) provided between the blood pressure measurement unit 20 and the control unit 23. The pressure signals acquired by the control unit 23 correspond to instantaneous values of the internal pressure of the radial artery TD. Accordingly, time series data of a blood pressure waveform can be obtained by acquiring a pressure signal at a time granularity and continuity that enable the blood pressure waveform of a single heartbeat to be obtained. The control unit 23 stores the pressure signals sequentially obtained from the pressure sensor 30 in the storage unit 27 along with information of the measurement times of the signals. The control unit 23 may store the acquired pressure signals as-is in the storage unit 27, or may store the pressure signals in the storage unit 27 after applying necessary signal processing to the pressure signals. The "necessary signal processing" may include processing for correcting the pressure signals so that the amplitude of the pressure signals matches a blood pressure value (e.g., an upper arm blood pressure), processing for reducing or removing noise from the pressure signals, or the like, for example.

Fig. 4 is a diagram illustrating a blood pressure waveform measured by the blood pressure measurement unit 20. The horizontal axis represents time, and the vertical axis represents blood pressure. The sampling frequency can be set as desired, but is preferably set to greater than or equal to 100 Hz in order to reproduce the shape characteristics of the waveform of a single heartbeat. Because the period of a single heartbeat is approximately one second, approximately 100 or more data points can be acquired in the waveform of a single heartbeat.

The blood pressure measurement unit 20 according to the present embodiment has advantages such as those described below.

A blood pressure waveform can be measured for each heartbeat. Thus for example, a variety of indicators related to blood pressure, heart condition, cardiovascular risk, and so on can be obtained on the basis of the shape characteristics of the blood pressure waveform. Additionally, the instantaneous value of the blood pressure can be monitored, which makes it possible to immediately detect blood pressure surges (sudden rises in blood pressure value), reliably detect blood pressure fluctuations and disturbances in the blood pressure waveform appearing only over extremely short amounts of time (one to several heartbeats), and so on.

Blood pressure monitors that are secured to the wrist or upper arm and measure blood pressure through the oscillometric method are in practical use as portable blood pressure monitors. However, a conventional portable blood pressure monitor can only measure a mean blood pressure value from fluctuations in the internal pressure of a cuff over several heartbeats, spanning several seconds to several tens of seconds, and thus cannot obtain time series data of a blood pressure waveform for each heartbeat, as is the case with the blood pressure measurement unit 20 according to the present embodiment.

The blood pressure waveform time series data can be recorded. When the blood pressure waveform time series data is acquired, a variety of indicators pertaining to blood pressure, heart condition, cardiovascular risk, and the like can be obtained by finding characteristics pertaining to changes in the blood pressure waveform over time, analyzing the frequency of the time series data and extracting specific frequency components, and so on, for example.

Because the device is configured as a portable (wearable) device, measurements place little burden on the user, and it is relatively easy to take continuous measurements for long periods of time, monitor blood pressure throughout the entire day, and so on. Furthermore, the portable form makes it possible not only to measure resting blood pressure, but also to measure changes in blood pressure during free movement (e.g., during daily activities, exercise, and so on). This in turn makes it possible to understand the effects of daily activities (sleeping, meals, commuting, work, taking medicine, and so on), exercise, and so on on blood pressure, for example.

Conventional products are devices of a type in which a blood pressure measurement unit is secured to the arm or wrist and the measurement is taken in a state of rest, and changes in blood pressure during daily activities, exercise, and so on cannot be measured, as with the biological information analyzing system 10 according to the present embodiment.

It is easy to link or combine the system with other sensors. For example, causal relationship evaluations or compound evaluations can be made using information obtained from other sensors (body movement, environment information such as temperature, other biological information such as SpO2 or breathing, and the like).

### Biological Information Analyzing Device

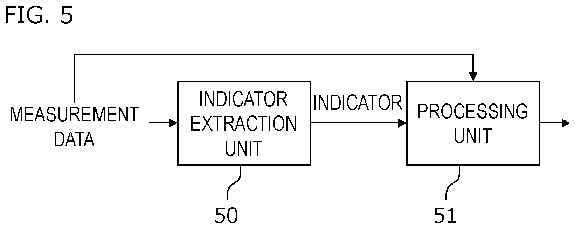

Fig. 5 is a block diagram illustrating processing performed by the biological information analyzing device 1. As illustrated in Fig. 5, the biological information analyzing device 1 includes an indicator extraction unit 50 and a processing unit 51. In the present embodiment, the processes of the indicator extraction unit 50 and the processing unit 51 may be realized by the control unit 23 executing necessary programs. These programs may be stored in the storage unit 27. When executing the necessary programs, the control unit 23 loads the programs in question, which are stored in the ROM or the storage unit 27, into the RAM. The control unit 23 then uses a CPU to interpret and execute the programs loaded into the RAM, and controls the various constituent elements. However, some or all the processing of the indicator extraction unit 50 and the processing unit 51 may be implemented by a circuit such as an ASIC, a FPGA, or the like. Alternatively, some or all of the processing of the indicator extraction unit 50 and the processing unit 51 may be implemented by a computer separate from the main unit 11 (e.g., a smartphone, a tablet terminal, a personal computer, a cloud server, or the like).

The indicator extraction unit 50 obtains, from the storage unit 27, the blood pressure waveform time series data measured continuously by the blood pressure measurement unit 20. The indicator extraction unit 50 extracts an indicator pertaining to characteristics of the blood pressure waveform, from the obtained blood pressure waveform time series data. Here, "characteristics of the blood pressure waveform" include shape characteristics of the blood pressure waveform for a single heartbeat, changes in the blood pressure waveform over time, a frequency component of the blood pressure waveform, and so on. The blood pressure waveform characteristics are not limited thereto, however. The extracted indicator is output to the processing unit 51. Because there are a variety of blood pressure waveform characteristics and indicators, the characteristics and indicators to be extracted can be designed and selected as appropriate in accordance with the purpose of the processing by the processing unit 51. The characteristics and indicators that can be extracted from the blood pressure waveform measurement data in the present embodiment will be described later.

When finding the indicator, the indicator extraction unit 50 can use the measurement data from the body movement measurement unit 21 and/or the measurement data from the environment measurement unit 22 in addition to the blood pressure waveform measurement data. Although not illustrated, measurement data from a sleep sensor, an SpO2 sensor, a breathing sensor (flow sensor), a blood sugar level sensor, or the like may be combined as well. Carrying out a compound analysis on multiple types of measurement data obtained from multiple types of sensors enables a higher level of information analysis to be carried out on the blood pressure waveform. For example, the blood pressure waveform data can be classified into user states, such as resting and active, times of high and low temperature, times of light and deep sleep, breathing and apnea, and so on. Alternatively, causal relationships, correlations, and so on among the measurement data can be evaluated, by extracting the effects of body movement, activity amounts and activity intensity, changes in temperature, how breathing or apnea manifests, and so on on blood pressure. Note that apnea includes obstructive apnea, central apnea, and mixed apnea.

The processing unit 51 receives the indicator extracted by the indicator extraction unit 50. The processing unit 51 carries out processing on the basis of the received indicator. A variety of processes are conceivable as the processes based on the indicator. For example, values of or changes in the extracted indicator may be provided to the user, a doctor, a nurse, or the like and used for health management, treatment, health guidance, and so on.

### Information Obtained from Blood Pressure Waveform

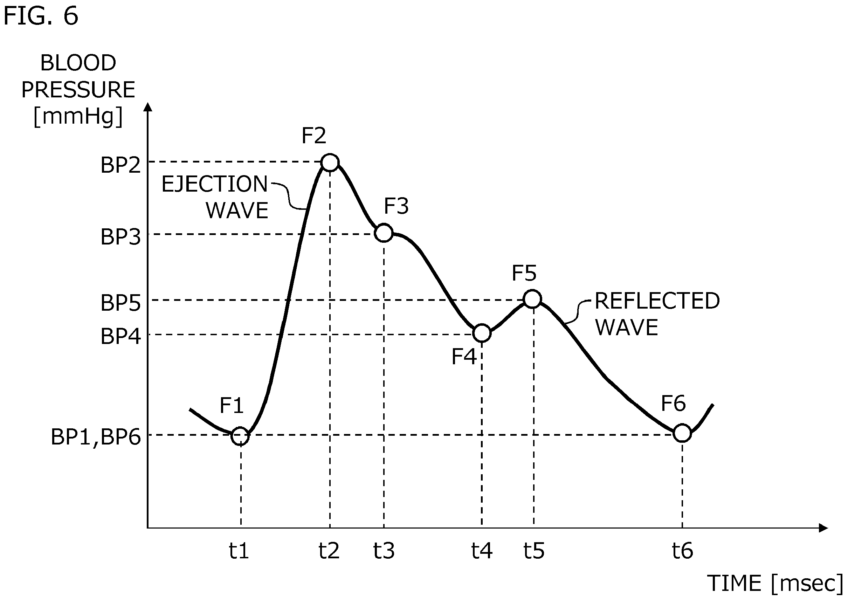

Fig. 6 is a diagram illustrating the waveform of a pressure pulse (a blood pressure waveform) in the radial artery, for a single heartbeat. The horizontal axis represents time t [msec], and the vertical axis represents blood pressure BP [mmHg].

The blood pressure waveform is a compound wave including an "ejection wave" produced when the heart contracts to expel blood and a "reflected wave" produced when the ejection wave is reflected by peripheral vessels, arterial branches, and so on. Examples of characteristic points that can be extracted from a blood pressure waveform corresponding to a single heartbeat are listed below.

- Point F1 is a point corresponding to the rise of the pressure pulse. The point F1 corresponds to an ejection start point of the heart, i.e., a point when the aortic valve opens.
- Point F2 is a point where the amplitude (pressure) of the ejection wave is maximum (a first peak).
- Point F3 is an inflection point appearing partway along the fall of the ejection wave due to the superposition of the reflected wave.
- Point F4 is a minimum point appearing between the ejection wave and the reflected wave, and is also called a "notch". This corresponds to a point when the aortic valve closes.
- Point F5 is a peak in the reflected wave appearing after point F4 (a second peak).
- Point F6 is the end point of the single heartbeat, and corresponds to the ejection start point of the next heartbeat, i.e., the starting point of the next heartbeat.

The indicator extraction unit 50 may use any algorithm to detect the characteristic points. For example, the indicator extraction unit 50 may extract a characteristic point (inflection point) of the blood pressure waveform by operating so as to find a nth-order differential waveforms of the blood pressure waveform and detect a zero crossing point thereof (for points F1, F2, F4, F5, and F6, this can be detected from a first-order differential waveform, and for point F3, from a second-order or fourth-order waveform). Alternatively, the indicator extraction unit 50 may identify the positions of the characteristic points by reading out a waveform pattern, in which characteristic points have been arranged in advance, from the storage unit 27, and fitting the blood pressure waveform in question to the waveform pattern.

By operating on the basis of the times t and the pressures BP of the above characteristic points F1 to F6, the indicator extraction unit 50 can obtain a variety of information (values, feature amounts, indicators, and the like) from the blood pressure waveform of a single heartbeat. The following provides representative examples of information that can be obtained from the blood pressure waveform. Note that tx and BPx represent the time and blood pressure, respectively, of a characteristic point Fx.

- pulse wave interval (heartbeat period) TA = t6 - t1
- heart rate PR = 1/TA
- pulse wave rise time UT = t2 - t1
- systole TS = t4 - t1
- diastole TD = t6 - t4
- reflected wave delay time = t3 - t1
- maximum blood pressure (systolic blood pressure) SBP = BP2
- minimum blood pressure (diastolic blood pressure) DBP = BP1
- mean blood pressure MAP = area of blood pressure waveform from t1 to t6 / heartbeat period TA
- systolic mean blood pressure = area of blood pressure waveform from t1 to t4 / systole TS
- diastolic mean blood pressure = area of blood pressure waveform from t4 to t6 / diastole TD
- pulse pressure PP = maximum blood pressure SBP - minimum blood pressure DBP
- late systolic pressure SBP2 = BP3
- AI (Augmentation Index) = (late systolic pressure SBP2 - minimum blood pressure DBP) / pulse pressure PP

Basic statistical amounts of this information (values, feature amounts, indicators) can also be used as indicators. The basic statistical amounts include representative values (mean values, median values, mode values, maximum values, minimum values, and the like) and dispersion (variance, standard deviation, coefficient of variation, and the like), for example. Changes over time in this information (values, characteristic values, indicators) can also be used as indicators.

By computing a plurality of pieces of beat information, the indicator extraction unit 50 can obtain an indicator called BRS (baroreflex sensitivity). This is an indicator expressing the capacity of blood pressure to regulate to a constant value. The spontaneous sequence method is an example of the calculation method. This is a method in which only a sequence in which the maximum blood pressure SBP and the pulse wave interval TA rise or fall in synchronization for three or more consecutive beats is extracted, the maximum blood pressure SBP and the pulse wave interval TA are plotted on a two-dimensional plane, and a slope obtained when a regression line is found through the least-squares method is defined as the BRS.

As described thus far, using the biological information analyzing system 10 according to the present embodiment makes it possible to obtain a variety of information from the blood pressure waveform data. However, the biological information analyzing system 10 need not include functions for obtaining all of the above-described information. It is acceptable to provide only the functions for obtaining the necessary information, in accordance with the configuration, user, purpose of usage, location of usage, and so on of the biological information analyzing system 10. Additionally, the configuration may be such that the functions are provided as program modules (application software), and functions can be added by installing the required program modules in the biological information analyzing system 10.

The following Example describes a specific example of the application of the biological information analyzing system 10.

### Example 1

To accurately ascertain a person's event occurrence risk from blood pressure fluctuations, the cause of the fluctuation, the way in which the fluctuation appears, and so on should be evaluated comprehensively. However, it is difficult to express the way in which a blood pressure fluctuation appears in a section where the blood pressure has fluctuated (called a "blood pressure fluctuation section" hereinafter) using only a simple maximum blood pressure value (SBP), minimum blood pressure value (DPB), and so on. Accordingly, in this Example, the blood pressure fluctuation section is detected from the blood pressure waveform time series data, and an indicator expressing the user's blood pressure fluctuation type is found on the basis of the cause of the blood pressure fluctuation in that section, a feature amount of the blood pressure fluctuation in that section, and so on. Turning the blood pressure fluctuation type into an indicator can be expected to make it possible to accurately estimate event occurrence risks on a user-by-user basis.

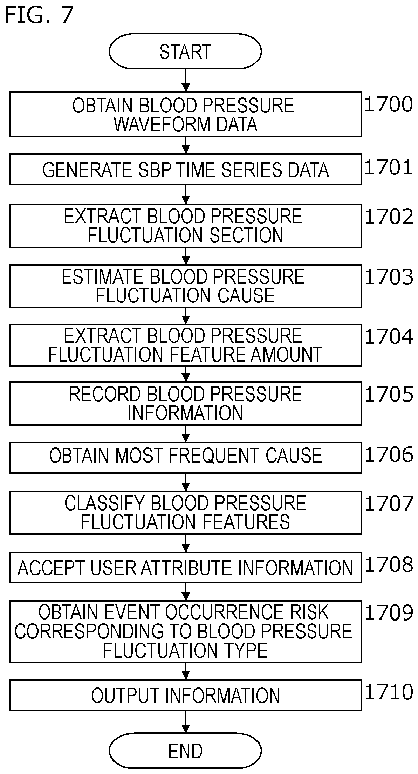

Fig. 7 is a flowchart illustrating processing according to this Example.

First, the indicator extraction unit 50 loads the blood pressure waveform time series data from the storage unit 27 (step 1700). For the purpose of ascertaining trends in the user's blood pressure fluctuation, it is preferable that the indicator extraction unit 50 obtain data for a sufficiently long period of time (e.g., one week to one month). Next, the indicator extraction unit 50 extracts a systolic blood pressure (SBP) value for each heartbeat, and the times thereof, from the blood pressure waveform for each heartbeat (step 1701). SBP time series data is obtained as a result. Although SBP is used in this Example, another feature amount may be used instead (e.g., diastolic blood pressure (DBP), mean arterial pressure (MAP), and so on).

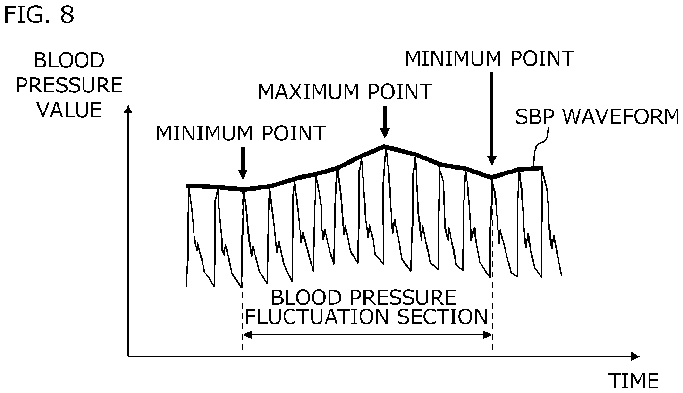

Next, the indicator extraction unit 50 analyzes the SBP time series data and extracts the blood pressure fluctuation section (step 1702). The method for extracting the blood pressure fluctuation section is not particularly limited. For example, as illustrated in Fig. 8, the indicator extraction unit 50 may detect a minimum point and maximum point of an SBP waveform obtained by connecting the SBPs in the SBP time series data (minimum points/maximum points caused by fine vibrations may be ignored in the SBP waveform at this time), and the period between two minimum points on either side of a given maximum point may be extracted as the blood pressure fluctuation section. Using this extraction method makes it possible to detect the blood pressure fluctuation section simply and automatically.

Next, the indicator extraction unit 50 estimates the cause of the blood pressure fluctuation in the blood pressure fluctuation section (step 1703). The cause of the blood pressure fluctuation may be estimated on the basis of information obtained from a sensor that detects the user's state (e.g., the body movement measurement unit 21, the environment measurement unit 22, or the like). For example, if it can be determined that the user is engaged in exercise, the user has stood up from a seated state, or the like on the basis of information obtained by the body movement measurement unit 21 at a time corresponding to the blood pressure fluctuation section, the indicator extraction unit 50 may estimate that the cause of the blood pressure fluctuation is "exercise", "standing change in attitude)", or the like. Additionally, if it can be determined that the outdoor temperature, indoor temperature, or the like has changed suddenly on the basis of information obtained by the environment measurement unit 22 at a time corresponding to the blood pressure fluctuation section, the indicator extraction unit 50 may estimate that the cause the blood pressure fluctuation is a "change in temperature". The "time corresponding to the blood pressure fluctuation section" may be a time within the blood pressure fluctuation section, or, if there is a time delay between the occurrence of the cause and the blood pressure fluctuation, may be a time prior to the blood pressure fluctuation section by a predetermined amount of time (the correspondence relationship between the blood pressure fluctuation section and the time of the cause of the blood pressure fluctuation may be changed as appropriate depending on the type of the cause of the blood pressure fluctuation). Furthermore, if the time of the blood pressure fluctuation section is at night, the indicator extraction unit 50 may estimate the occurrence of apnea as the cause of the blood pressure fluctuation. Alternatively, the cause of the blood pressure fluctuation may be entered by the user him/herself.

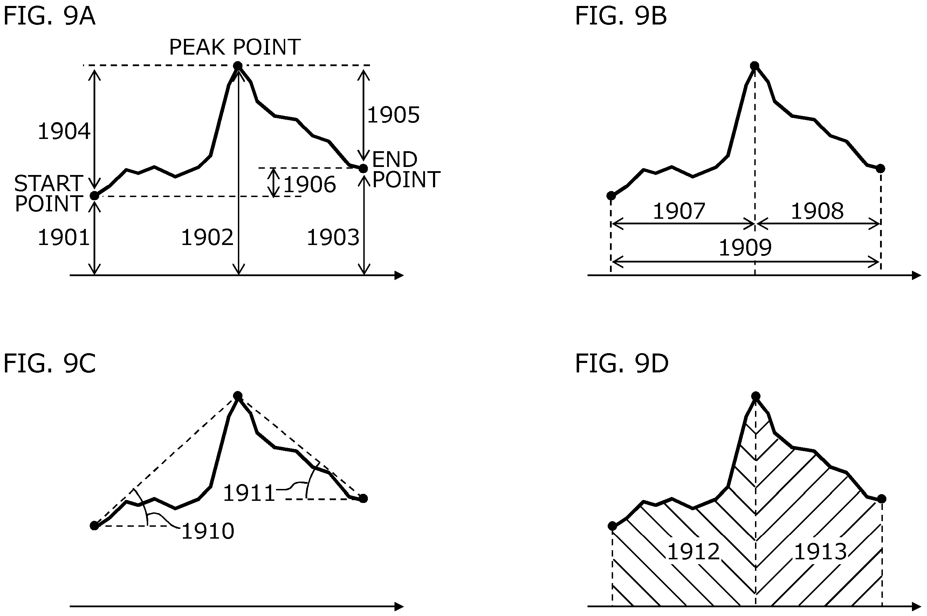

Next, the indicator extraction unit 50 extracts various types of feature amounts from the SBP data in the blood pressure fluctuation section (step 1704). The types and number of the extracted feature amounts are not particularly limited. For example, a basic statistical amount of the SBP in the blood pressure fluctuation section, the SBP waveform in the blood pressure fluctuation section, and so on are feature amounts. Basic statistical amounts include mean, median, maximum value, minimum value, a difference between a maximum value and a minimum value, standard deviation, distortion, sharpness, and so on. As illustrated in Figs. 9A to 9D, the waveform feature amounts include: an SBP value 1901 at a start point of the blood pressure fluctuation section; an SBP value 1902 at a peak point; an SBP value 1903 at an end point; an SBP change amount 1904 between the start point and the peak point; an SBP change amount 1905 between the peak point and the end point; an SBP change amount 1906 between the start point and the end point; a time 1907 between the start point and the peak point; a time 1908 between the peak point and the end point; a time 1909 between the start point and the end point; a rise angle 1910 from the start point to the peak point; a fall angle 1911 from the peak point to the end point; an SBP average from the start point to the end point; an SBP standard deviation from the start point to the peak point; an SBP standard deviation from the peak point to the end point; an SBP standard deviation from the start point to the end point; an area 1912 of the SBP waveform from the start point to the peak point; an area 1913 of the SBP waveform from the peak point to the end point; an area of the SBP waveform from the start point to the end point (the sum of the areas 1912 and 1913); a minimum point in the SBP waveform; and so on.

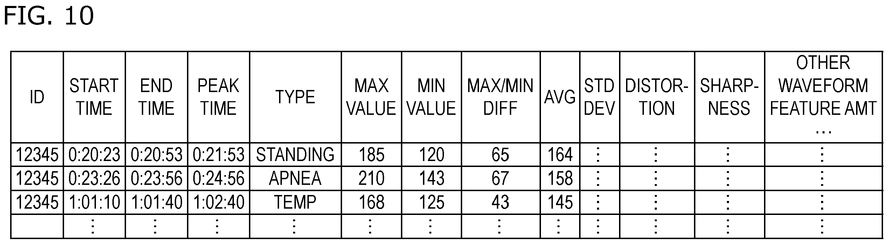

Next, the indicator extraction unit 50 records the cause of the blood pressure fluctuation and the blood pressure fluctuation feature amounts in the storage unit 27 as information pertaining to the blood pressure fluctuation section (step 1705). If a plurality of blood pressure fluctuation sections have been extracted from the blood pressure waveform data, the processing of steps 1703 to 1705 are repeated for each blood pressure fluctuation section. Fig. 10 is an example of information pertaining to the user's blood pressure fluctuation section stored in the storage unit 27 (called simply "blood pressure information" hereinafter). Information of the cause of the blood pressure fluctuation and the blood pressure fluctuation feature amounts is recorded for each instance of the blood pressure fluctuation. "ID" is an identifier for identifying the user.

Next, the indicator extraction unit 50 analyzes the blood pressure information and finds the most frequent cause of the blood pressure fluctuation (most frequent cause) (step 1706). For example, if in the blood pressure information for a given user a blood pressure fluctuation cause of "standing" is 50%, a blood pressure fluctuation cause of "exercise" is 10%, a blood pressure fluctuation cause of "apnea" is 20%, and a blood pressure fluctuation cause of "temperature change" is 20%, the most frequent cause for that user is "standing".

Next, the indicator extraction unit 50 calculates the blood pressure fluctuation feature amounts on the basis of the blood pressure information (step 1707). Specifically, the indicator extraction unit 50 refers to a class database in which trends of the blood pressure fluctuation feature amounts are registered as a plurality of different groups and evaluates the similarities between the user's blood pressure fluctuation feature amount and the blood pressure fluctuation feature amount in each group, and determines whether the user's blood pressure fluctuation feature amount conforms to the trend of the blood pressure fluctuation feature amount in any of the groups. Although the class database is stored in the storage unit 27 in this Example, the class database may be present in storage, a server, or the like external to the biological information analyzing device 1 (e.g., a cloud server, online storage, or the like).

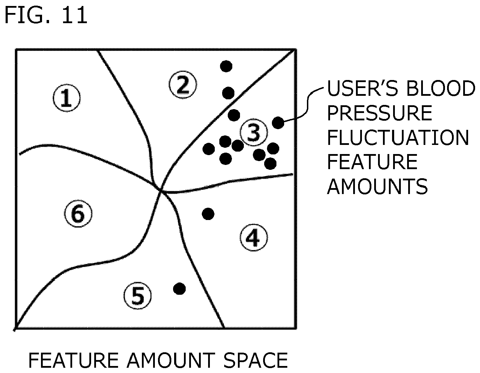

Fig. 11 conceptually illustrates the processing of step 1707. Blood pressure fluctuation feature amount data obtained from multiple subjects is registered in the class database, and that data is divided into K groups (also called "classes") in advance on the basis of the similarity of the feature amounts. This grouping is carried out through a known clustering method such as hierarchical clustering, the K-Means method, or the like. Fig. 11 schematically illustrates a feature amount space having been divided into six groups (classes). The indicator extraction unit 50 maps the user's blood pressure fluctuation feature amount data onto the feature amount space. In the example of Fig. 11, the highest number of data points have been mapped onto the area of group 3, and thus the user's blood pressure fluctuation feature amount is classified as "group 3". The blood pressure fluctuation feature amount classification (trend) will be called a "fluctuation classification" hereinafter.

Next, the indicator extraction unit 50 accepts attribute information (age, sex, presence/absence of illness, and the like) of the user (step 1708). For example, the user may be allowed to enter the attribute information him/herself using the input unit 24.

Through the processing described above, two indicators are obtained, namely a fluctuation cause of "standing" and a fluctuation classification of "group 3", for the user's blood pressure fluctuation. These indicators can be said to express the user's blood pressure fluctuation type. The attribute information is also obtained as information pertaining to the user's event occurrence risk.

Next, the processing unit 51 refers to the risk database, and obtains information of a risk corresponding to the user's blood pressure fluctuation type (step 1709). Although the risk database is stored in the storage unit 27 in this Example, the risk database may be present in storage, a server, or the like external to the biological information analyzing device 1 (e.g., a cloud server, online storage, or the like).

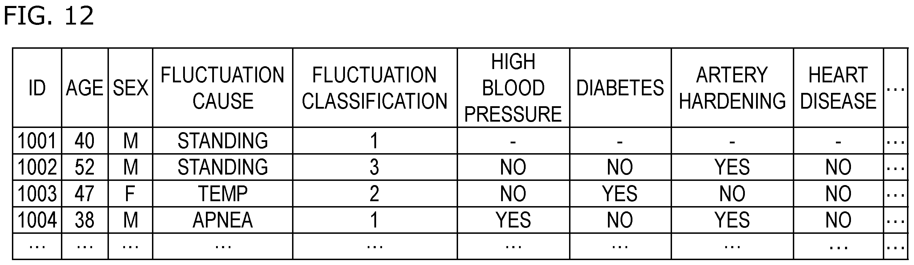

Fig. 12 illustrates an example of the risk database. Data of attribute information such as age and sex, blood pressure fluctuation types (fluctuation causes, fluctuation classifications), and risk information (pre-existing conditions and the like), obtained from multiple subjects, is registered in the risk database in advance. For example, it can be seen from registered data having an ID of 1003 that the subject is a 47-year-old woman, whose blood pressure fluctuates easily in response to changes in "temperature", whose fluctuation classification is group 2, and who has diabetes as a pre-existing condition.

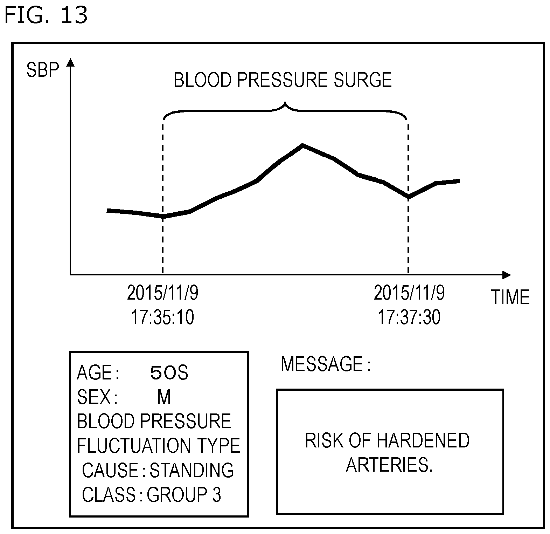

In step 1709, the processing unit 51 searches the risk database for the registered data closest to the user's attribute information and blood pressure fluctuation type (fluctuation cause, fluctuation classification) obtained in steps 1706 to 1708, and reads out the risk information in the registered data that is found. In step 1710, the processing unit 51 presents information such as the users blood pressure fluctuation type, event occurrence risk, and so on on the basis of the read-out risk information. Fig. 13 is an example of an information display screen output to the output unit 25 (a display device) by the processing unit 51. In this example, a message such as "risk of hardened arteries" is output along with information such as the SBP waveform (blood pressure surge waveform) in the blood pressure fluctuation section, the user's attribute information and blood pressure fluctuation type (fluctuation cause, fluctuation classification), and the like.

According to the configuration described thus far, the user's blood pressure fluctuation type, risk, and so on are determined on the basis of measurement data in a section where the blood pressure has fluctuated, and that information is then output. Accordingly, the user can take appropriate measures in light of his/her own characteristics, risk, and so on, and can therefore prevent the occurrence of a serious event. If the system is used by a doctor, a nurse, or the like, that person can refer to the analysis information, such as the user's (patient's) blood pressure fluctuation type and risk, and then carry out treatment, guidance, and so on appropriate for the user's characteristics, risk, and so on.

Note that the configurations in the above-described embodiment and Example are merely specific examples of the present invention, and are not intended to limit the scope of the present invention, which is defined by the appended claims.

### INDEX TO THE REFERENCE NUMERALS

1... biological information analyzing device
2... measurement unit
10... biological information analyzing system
11... main unit
12... belt
20... blood pressure measurement unit
21... body movement measurement unit
22... environment measurement unit
23... control unit
24... input unit
25... output unit
26... communication unit
27... storage unit
30... pressure sensor
31... compression mechanism
300... pressure detection element
50... indicator extraction unit
51... processing unit

## Claims

1. A biological information analyzing device (1) comprising:
an indicator extraction unit (50) configured to obtain data of a blood pressure fluctuation section, which is a section in which a blood pressure fluctuation has occurred, from time series data of a blood pressure waveform measured continuously by a sensor (30), which is configured to be worn on a user's body and can non-invasively measure a blood pressure waveform for each of heartbeats, and extract an indicator expressing a blood pressure fluctuation type of the user from the data of the blood pressure fluctuation section; and
a processing unit (51) configured to output the indicator expressing the blood pressure fluctuation type extracted by the indicator extraction unit (50), wherein the indicator extraction unit (50) is configured to calculate time series data of a systolic blood pressure for each heartbeat from the time series data of the blood pressure waveform, and extract, as the data of the blood pressure fluctuation section, data of a period between two minimum points on either side of a maximum point in the time series data of the systolic blood pressure.

2. The biological information analyzing device (1) according to claim 1,
wherein the indicator extraction unit is configured to extract a blood pressure fluctuation feature amount from the data of the blood pressure fluctuation section, and take a trend in the extracted blood pressure fluctuation feature amount as one indicator expressing the blood pressure fluctuation type.

3. The biological information analyzing device (1) according to claim 2,
wherein the indicator extraction unit (50) is configured to classify the user's blood pressure fluctuation feature amount by referring to a class database in which trends of the blood pressure fluctuation feature amount are registered as a plurality of different groups and determine whether the blood pressure fluctuation feature amount extracted from the data of the blood pressure fluctuation section conforms to the trend of the blood pressure fluctuation feature amount in any of the plurality of groups.

4. The biological information analyzing device (1) according to claim 3, further comprising:
a storage unit (27) configured to store the class database in advance.

5. The biological information analyzing device (1) according to any one of claims 1 to 4,
wherein the indicator extraction unit (50) is configured to estimate a cause of the blood pressure fluctuation in the blood pressure fluctuation section, and take the estimated cause as one indicator expressing the blood pressure fluctuation type.

6. The biological information analyzing device (1) according to claim 5,
wherein the indicator extraction unit (50) is configured to estimate the cause of the blood pressure fluctuation in the blood pressure fluctuation section on the basis of information pertaining to a state of the user detected at a time corresponding to the blood pressure fluctuation section by a second sensor configured to detect the state of the user.

7. The biological information analyzing device (1) according to any one of claims 1 to 6,
wherein the indicator extraction unit (50) is configured to obtain risk information corresponding to the user's blood pressure fluctuation type from a risk database in which the blood pressure fluctuation type is associated with the risk information; and
the processing unit (51) is configured to output the risk information obtained by the indicator extraction unit (50).

8. The biological information analyzing device (1) according to claim 7, further comprising:
a storage unit (27) that is configured to store the risk database in advance.

9. A biological information analyzing system (10) comprising:
a sensor, which is configured to be worn on a user's body and can non-invasively measure a blood pressure waveform for each of heartbeats; and
the biological information analyzing device (1) according to any one of claims 1 to 8, the biological information analyzing device (1) being configured to analyze biological information using data of the blood pressure waveform measured continuously by the sensor (30).

10. A program causing a processor to function as the indicator extraction unit (50) and the processing unit (51) of the biological information analyzing device (1) according to any one of claims 1 to 8.

11. A biological information analyzing method comprising the following steps performed by an indicator extraction unit:
a step of obtaining data of a blood pressure fluctuation section, which is a section in which a blood pressure fluctuation has occurred, from time series data of a blood pressure waveform measured continuously by a sensor (30), which is configured to be worn on a user's body and can non-invasively measure a blood pressure waveform for each of heartbeats;
a step of calculating time series data of a systolic blood pressure for each heartbeat;
a step of extracting, as data of blood pressure fluctuation section which is a section in which blood pressure fluctuation has occurred, data of a period between two minimum points on either side of a maximum point in the time series data of the systolic blood pressure;
a step of extracting an indicator expressing a blood pressure fluctuation type of the user from the data of the blood pressure fluctuation section; an further comprising the following step performed by a processing unit:
a step of outputting the extracted indicator expressing the blood pressure fluctuation type.

## Patentansprüche

1. Vorrichtung (1) zur Analyse biologischer Informationen, umfassend:
eine Indikatorextraktionseinheit (50), die dafür eingerichtet ist, Daten eines Blutdruckschwankungsabschnitts, der ein Abschnitt ist, in dem eine Blutdruckschwankung aufgetreten ist, aus Zeitreihendaten einer Blutdruckwellenform zu erhalten, die kontinuierlich durch einen Sensor (30) gemessen wird, der dafür eingerichtet ist, am Körper eines Benutzers getragen zu werden, und der nicht-invasiv eine Blutdruckwellenform für jeden von Herzschlägen messen kann, und einen Indikator, der einen Blutdruckschwankungstyp des Benutzers ausdrückt, aus den Daten des Blutdruckschwankungsabschnitts zu extrahieren; und
eine Verarbeitungseinheit (51), die dafür eingerichtet ist, den Indikator auszugeben, der den durch die Indikatorextraktionseinheit (50) extrahierten Blutdruckschwankungstyp ausdrückt,
wobei die Indikatorextraktionseinheit (50) dafür eingerichtet ist, Zeitreihendaten eines systolischen Blutdrucks für jeden Herzschlag aus den Zeitreihendaten der Blutdruckwellenform zu berechnen und, als die Daten des Blutdruckschwankungsabschnitts, Daten eines Zeitraums zwischen zwei Minimumpunkten auf jeder Seite eines Maximalpunkts in den Zeitreihendaten des systolischen Blutdrucks zu extrahieren.

2. Vorrichtung (1) zur Analyse biologischer Informationen nach Anspruch 1,
wobei die Indikatorextraktionseinheit dafür eingerichtet ist, eine Blutdruckschwankungsmerkmalsmenge aus den Daten des Blutdruckschwankungsabschnitts zu extrahieren und eine Tendenz in der extrahierten
Blutdruckschwankungsmerkmalsmenge als einen Indikator zu nehmen, der den Blutdruckschwankungstyp ausdrückt.

3. Vorrichtung (1) zur Analyse biologischer Informationen nach Anspruch 2,
wobei die Indikatorextraktionseinheit (50) dafür eingerichtet ist, die Blutdruckschwankungsmerkmalsmenge des Benutzers durch Nachschlagen in einer Klassendatenbank, in der Tendenzen der Blutdruckschwankungsmerkmalsmenge als mehrere verschiedene Gruppen registriert sind, zu klassifizieren und zu bestimmen, ob die aus den Daten des Blutdruckschwankungsabschnitts extrahierte Blutdruckschwankungsmerkmalsmenge mit der Tendenz der Blutdruckschwankungsmerkmalsmenge in einer der mehreren Gruppen übereinstimmt.

4. Vorrichtung (1) zur Analyse biologischer Informationen nach Anspruch 3, des Weiteren umfassend:
eine Speichereinheit (27), die dafür eingerichtet ist, die Klassendatenbank im Voraus zu speichern.

5. Vorrichtung (1) zur Analyse biologischer Informationen nach einem der Ansprüche 1 bis 4,
wobei die Indikatorextraktionseinheit (50) dafür eingerichtet ist, eine Ursache der Blutdruckschwankung in dem Blutdruckschwankungsabschnitt zu schätzen und die geschätzte Ursache als einen Indikator zu nehmen, der den Blutdruckschwankungstyp ausdrückt.

6. Vorrichtung (1) zur Analyse biologischer Informationen nach Anspruch 5,
wobei die Indikatorextraktionseinheit (50) dafür eingerichtet ist, die Ursache der Blutdruckschwankung in dem Blutdruckschwankungsabschnitt auf der Grundlage von Informationen zu schätzen, die sich auf einen Zustand des Benutzers beziehen, der zu einer Zeit, die dem Blutdruckschwankungsabschnitt entspricht, durch einen zweiten Sensor detektiert wird, der dafür eingerichtet ist, den Zustand des Benutzers zu detektieren.

7. Vorrichtung (1) zur Analyse biologischer Informationen nach einem der Ansprüche 1 bis 6,
wobei die Indikatorextraktionseinheit (50) dafür eingerichtet ist, Risikoinformationen, die dem Blutdruckschwankungstyp des Benutzers entsprechen, aus einer Risikodatenbank zu erhalten, in welcher der Blutdruckschwankungstyp mit den Risikoinformationen verknüpft ist; und
die Verarbeitungseinheit (51) dafür eingerichtet ist, die durch die Indikatorextraktionseinheit (50) erhaltenen Risikoinformationen auszugeben.

8. Vorrichtung (1) zur Analyse biologischer Informationen nach Anspruch 7, des Weiteren umfassend:
eine Speichereinheit (27), die dafür eingerichtet ist, die Risikodatenbank im Voraus zu speichern.

9. System (10) zur Analyse biologischer Informationen, umfassend:
einen Sensor, der dafür eingerichtet ist, am Körper des Benutzers getragen zu werden, und der nicht-invasiv eine Blutdruckwellenform für jeden von Herzschlägen messen kann; und
die Vorrichtung (1) zur Analyse biologischer Informationen nach einem der Ansprüche 1 bis 8, wobei die Vorrichtung (1) zur Analyse biologischer Informationen dafür eingerichtet ist, biologische Informationen unter Verwendung von Daten der Blutdruckwellenform zu analysieren, die kontinuierlich durch den Sensor (30) gemessen wird.

10. Programm, das einen Prozessor veranlasst, als die Indikatorextraktionseinheit (50) und die Verarbeitungseinheit (51) der Vorrichtung (1) zur Analyse biologischer Informationen nach einem der Ansprüche 1 bis 8 zu dienen.

11. Verfahren zur Analyse biologischer Informationen, das die folgenden Schritte umfasst, die durch eine Indikatorextraktionseinheit durchgeführt werden:
einen Schritt des Erhaltens von Daten eines Blutdruckschwankungsabschnitts, der ein Abschnitt ist, in dem eine Blutdruckschwankung aufgetreten ist, aus Zeitreihendaten einer Blutdruckwellenform, die kontinuierlich durch einen Sensor (30) gemessen wird, der dafür eingerichtet ist, am Körper eines Benutzers getragen zu werden, und der nicht-invasiv eine Blutdruckwellenform für jeden von Herzschlägen messen kann; und
einen Schritt des Berechnens von Zeitreihendaten eines systolischen Blutdrucks für jeden Herzschlag;
einen Schritt des Extrahierens, als Daten des Blutdruckschwankungsabschnitts, der ein Abschnitt ist, in dem eine Blutdruckschwankung aufgetreten ist, von Daten eines Zeitraums zwischen zwei Minimumpunkten auf jeder Seite eines Maximalpunkts in den Zeitreihendaten des systolischen Blutdrucks;
einen Schritt des Extrahierens eines Indikators, der einen Blutdruckschwankungstyp des Benutzers ausdrückt, aus den Daten des Blutdruckschwankungsabschnitts; und
des Weiteren den folgenden Schritt umfasst, der durch eine Verarbeitungseinheit durchgeführt wird:
einen Schritt des Ausgebens des extrahierten Indikators, der den Blutdruckschwankungstyp ausdrückt.

## Revendications

1. Dispositif d'analyse d'informations biologiques (1) comprenant :
une unité d'extraction d'indicateur (50) configurée pour obtenir des données d'une section de fluctuation de pression artérielle, qui est une section dans laquelle s'est produite une fluctuation de pression artérielle, à partir de données de séries chronologiques d'une forme d'onde de pression artérielle mesurée en continu par un capteur (30) qui est conçu pour être porté sur le corps d'un utilisateur et qui peut mesurer de façon non effractive une forme d'onde de pression artérielle pour chacun des battements de cœur, et extraire un indicateur exprimant un type de fluctuation de pression artérielle de l'utilisateur, des données de la section de fluctuation de pression artérielle ; et
une unité de traitement (51) configurée pour délivrer l'indicateur exprimant le type de fluctuation de pression artérielle extrait par l'unité d'extraction d'indicateur (50),
dans lequel l'unité d'extraction d'indicateur (50) est configurée pour calculer des données de séries chronologiques d'une pression artérielle systolique pour chaque battement de cœur, à partir des données de séries chronologiques de la forme d'onde de pression artérielle, et pour extraire, en tant que données de la section de fluctuation de pression artérielle, les données d'une période comprise entre deux points minimaux de chaque côté d'un point maximal des données de séries chronologiques de la pression artérielle systolique.

2. Dispositif d'analyse d'informations biologiques (1) selon la revendication 1,
dans lequel l'unité d'extraction d'indicateur est configurée pour extraire une grandeur caractéristique de fluctuation de pression artérielle, des données de la section de fluctuation de pression artérielle, et pour prendre une tendance de la grandeur caractéristique de fluctuation de pression artérielle extraite comme indicateur exprimant le type de fluctuation de pression artérielle.

3. Dispositif d'analyse d'informations biologiques (1) selon la revendication 2,
dans lequel l'unité d'extraction d'indicateur (50) est configurée pour classer la grandeur caractéristique de fluctuation de pression artérielle de l'utilisateur en se référant à une base de données de classes dans laquelle les tendances de la grandeur caractéristique de fluctuation de pression artérielle sont enregistrées sous la forme d'une pluralité de groupes différents et pour déterminer si la grandeur caractéristique de fluctuation de pression artérielle extraite des données de la section de fluctuation de pression artérielle est conforme à la tendance de la grandeur caractéristique de fluctuation de pression artérielle d'un groupe quelconque de la pluralité de groupes.

4. Dispositif d'analyse d'informations biologiques (1) selon la revendication 3, comprenant en outre :
une unité de stockage (27) conçue pour stocker à l'avance la base de données de classes.

5. Dispositif d'analyse d'informations biologiques (1) selon l'une quelconque des revendications 1 à 4,
dans lequel l'unité d'extraction d'indicateur (50) est configurée pour estimer une cause de la fluctuation de pression artérielle dans la section de fluctuation de pression artérielle, et pour prendre la cause estimée comme indicateur exprimant le type de fluctuation de pression artérielle.

6. Dispositif d'analyse d'informations biologiques (1) selon la revendication 5,
dans lequel l'unité d'extraction d'indicateur (50) est configurée pour estimer la cause de la fluctuation de pression artérielle dans la section de fluctuation de pression artérielle sur la base d'informations se rapportant à un état de l'utilisateur, détecté à un instant correspondant à la section de fluctuation de pression artérielle, par un second capteur conçu pour détecter l'état de l'utilisateur.

7. Dispositif d'analyse d'informations biologiques (1) selon l'une quelconque des revendications 1 à 6,
dans lequel l'unité d'extraction d'indicateur (50) est configurée pour obtenir des informations de risques correspondant au type de fluctuation de pression artérielle de l'utilisateur, à partir d'une base de données de risques dans laquelle le type de fluctuation de pression artérielle est associé aux informations de risques ; et
l'unité de traitement (51) est configurée pour délivrer les informations de risques obtenues par l'unité d'extraction d'indicateur (50).

8. Dispositif d'analyse d'informations biologiques (1) selon la revendication 7, comprenant en outre :
une unité de stockage (27) qui est conçue pour stocker à l'avance la base de données de risques.

9. Système d'analyse d'informations biologiques (10) comprenant :
un capteur qui est conçu pour être porté sur le corps d'un utilisateur et qui peut mesurer de façon non effractive une forme d'onde de pression artérielle pour chacun des battements de cœur ; et
le dispositif d'analyse d'informations biologiques (1) selon l'une quelconque des revendications 1 à 8, le dispositif d'analyse d'informations biologiques (1) étant conçu pour analyser des informations biologiques à l'aide de données de la forme d'onde de pression artérielle mesurée en continu par le capteur (30).

10. Programme faisant fonctionner un processeur comme l'unité d'extraction d'indicateur (50) et l'unité de traitement (51) du dispositif d'analyse d'informations biologiques (1) selon l'une quelconque des revendications 1 à 8.

11. Procédé d'analyse d'informations biologiques comprenant les étapes suivantes exécutées par une unité d'extraction d'indicateur :
une étape consistant à obtenir des données d'une section de fluctuation de pression artérielle, qui est une section dans laquelle s'est produite une fluctuation de pression artérielle, à partir de données de séries chronologiques d'une forme d'onde de pression artérielle mesurée en continu par un capteur (30) qui est conçu pour être porté sur le corps d'un utilisateur et qui peut mesurer de façon non effractive une forme d'onde de pression artérielle pour chacun des battements de cœur ;
une étape consistant à calculer des données de séries chronologiques d'une pression artérielle systolique pour chaque battement de cœur ;
une étape consistant à extraire, en tant que données de section de fluctuation de pression artérielle, qui est une section dans laquelle s'est produite une fluctuation de pression artérielle, les données d'une période comprise entre deux points minimaux de chaque côté d'un point maximal des données de séries chronologiques de la pression artérielle systolique ;
une étape consistant à extraire un indicateur exprimant un type de fluctuation de pression artérielle de l'utilisateur, des données de la section de fluctuation de pression artérielle ;
et comprenant en outre l'étape suivante exécutée par une unité de traitement :
une étape consistant à délivrer l'indicateur extrait exprimant le type de fluctuation de pression artérielle.
